(19) Europäisches Patentamt
European Patent Office
Office européen des brevets

(11) **EP 2 776 959 B1**

(12) **EUROPEAN PATENT SPECIFICATION**

(45) Date of publication and mention
of the grant of the patent:
**11.08.2021 Bulletin 2021/32**

(51) Int Cl.:
**G16H 50/50** *(2018.01)*      **G16H 50/20** *(2018.01)*

(21) Application number: **12795177.0**

(22) Date of filing: **09.11.2012**

(86) International application number:
**PCT/US2012/064298**

(87) International publication number:
**WO 2013/071012 (16.05.2013 Gazette 2013/20)**

(54) **PERSONALIZED STRATEGIC CANCER TREATMENT**

STRATEGISCHE PERSONALISIERTE KREBSBEHANDLUNG

TRAITEMENT STRATÉGIQUE PERSONNALISÉ CONTRE LE CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.11.2011 US 201161558902 P**
**02.12.2011 US 201161566396 P**
**02.08.2012 US 201261678790 P**

(43) Date of publication of application:
**17.09.2014 Bulletin 2014/38**

(73) Proprietor: **Beckman, Robert**
**Blue Bell, Pennsylvania 19422 (US)**

(72) Inventor: **Beckman, Robert**
**Blue Bell, Pennsylvania 19422 (US)**

(74) Representative: **Purdylucey Intellectual Property**
**6-7 Harcourt Terrace**
**D02 FH73 Dublin 2 (IE)**

(56) References cited:
**WO-A1-2009/155009**

• R. A. Beckman ET AL: "Impact of genetic dynamics and single-cell heterogeneity on development of nonstandard personalized medicine strategies for cancer", PROCEEDINGS OF THE NATIONAL ACADEMY OF SCIENCES OF THE UNITED STATES OF AMERICA, vol. 109, no. 36, 13 August 2012 (2012-08-13), pages 14586-14591, XP055516013, ISSN: 0027-8424, DOI: 10.1073/pnas.1203559109

• R. A. Beckman ET AL: "Impact of genetic dynamics and single-cell heterogeneity on development of nonstandard personalized medicine strategies for cancer (Supporting Information)", PROCEEDINGS OF THE NATIONAL ACADEMY OF SCIENCES OF THE UNITED STATES OF AMERICA, vol. 109, no. 36, 13 August 2012 (2012-08-13), pages 1-8, XP055516017, ISSN: 0027-8424, DOI: 10.1073/pnas.1203559109

**Description**

Background

This invention relates to personalized strategic cancer treatment.

In conventional cancer treatment regimens medical practitioners treat patients with one or more cytotoxic chemotherapy drugs with the goal of reducing or eliminating an overall population of cancerous cells in one or more cancerous tumors. Such treatments are typically non-specific in that they kill any rapidly dividing cells (e.g., by damaging the DNA of the cells), including non-cancerous cells which happen to be rapidly dividing at the time of treatment. These treatments regimens are somewhat effective, in some cases prolonging the life of a patient and in occasional cases, completely curing a patient. However, the side effects due to the toxicity of these treatments can range from unpleasant to life threatening.

Some conventional cancer treatment regimens are personalized to provide a more effective, targeted therapy with fewer side effects by tailoring treatment regimens to address heterogeneity between individual cancers ("personalized cancer medicine"). For example, in practice, personalized cancer treatment regimens are designed by extensively characterizing the molecular makeup of a patient's tumor. Based on the unique molecular features of the patient's tumor, a specific medicine is applied, that was originally designed with precisely that type of tumor in mind. These treatment regimens are somewhat effective and are often most effective when used in combination with traditional chemotherapy. Personalized cancer treatment regimens can be especially effective in cases where the tumors to be treated are genetically simple (e.g., chronic myelogenous leukemia, b-raf mutated melanoma). The side effects of personal cancer treatment regimens are typically lesser but can still be significant.

There are a number of factors which can make it difficult to create an extremely effective personalized cancer treatment regimen. One factor is that cancerous tumors typically include heterogeneous cell types. For example, there are roughly 30,000 genes in the human genome with at least 100 genes know to be drivers of cancer. A cancerous tumor can include 20,000 to 30,000 mutations with no one mutation existing in more than a fraction of tumors. Furthermore, interactions between genes are numerous, complex, and in many cases, unknown. These factors can lead not only to heterogeneity *between* tumors (addressed by current personalized medicine paradigms), but by heterogeneity *within* tumors, between individual cells. Furthermore, it can be difficult to gain access to tumor tissue such that the tumor tissue can be genetically profiled. For example, some tumors cannot be biopsied without causing irreversible damage to the patient.

Dynamic resistance to therapy has been shown for many tumor types and by a variety of genetic and nongenetic mechanisms. In non-small cell lung cancer (NSCLC) treated with erlotinib or gefitinib, resistance mutations occur, most commonly in the target EGF receptor (EGFR). Other resistance mechanisms include activation of parallel signaling pathways, such as cMet, through amplification, which is, at times, ligand-induced. Importantly, when erlotinib or gefitinib resistance develops, the drug's withdrawal may trigger tumor rebound, suggesting the persistence of a sensitive subpopulation below the detection limit. Resistance to crizotinib, a drug targeted to a unique fusion protein involving the anaplastic lymphoma kinase in NSCLC, has been documented due to mutations in the target, amplification of the target, loss of the original translocation leading to the fusion protein, increased signaling in the EGFR pathway (including 1 EGFR activating mutation), c-Kit amplification, and KRAS mutation, sometimes with more than one resistance mechanism in the same patient. In chronic myelogenous leukemia, most therapeutic resistance is due to mutation in the targeted BCR-ABL fusion protein, and combinations may be important to delay the emergence of multiply resistant cells. Nongenetic resistance mechanisms occur in tumors and may be immediate because they are wired into feedback loops in signaling pathways. Recent examples include resistance to vemurafenib in colorectal cancer cells and to PI3-kinase inhibitors via up-regulation of upstream signaling pathways.

WO 2009/155009 describes D1 considers different combinations of agents that may be used, as represented by a vector $(u_k)$ for $k \in 1$ to $M$. Each variable $u_k$, referred to as a "perturbation variable", "indicates an externally controlled perturbation agent that might affect one or more relevant properties of the biological system." (see para. [0043]). "In some embodiments, values for the perturbation variables ... refer to drug concentrations." (see para. [0061]). If a single drug is selected, only one element of the *u* vector is non-zero, and that in general, the number of non-zero entries of the *u* vector corresponds to the number of drugs being combined. WO 2009/155009 only describes the application of a fixed "perturbation vector", *u*, and considers the time evolution of a dynamical system with that vector as its input.

Given these dynamics, there is a need to take possible future states into account, perhaps thinking several therapeutic maneuvers ahead.

Summary

In one aspect, in general, a new approach to personalized cancer therapy makes use of a mathematical model that incorporates genetic evolutionary dynamics and single-cell heterogeneity. Analyses of an illustrative case and a virtual clinical trial of over 3 million evaluable "patients" demonstrate that augmented (and sometimes counterintuitive) nonstandard personalized medicine strategies may lead to superior patient outcomes compared with the current personalized medicine approach. Current personalized medicine generally matches therapy to a tumor molecular profile at diagnosis and at tumor relapse or progression, generally focusing on the average, static, and current properties of the sample. In some examples, nonstandard strategies additionally consider minor subclones, dynamics, and predicted future tumor states. The new approach provides a way to systematically evaluate nonstandard personalized medicine strategies.

In another aspect, in general, an approach to determining a specific treatment plan for a subject uses of a prediction of time evolution of sub-populations of cells with different types of resistance to a set of therapeutic agents based at least in part of a sampling or measurement from the subject (e.g., tissue, bodily fluid, scan) and determines a therapeutic schedule for administration of selected ones of the agents according to a criterion that is based at least in part on a factor that depends on evolution of one or more sub-population.

In another aspect, in general, a method for treatment selection includes accepting data characterizing populations of a plurality of cell states of a sample (e.g., tissue, blood) from a subject. An effectiveness of each of a plurality of treatment strategies is then determined. Each treatment strategy represents a selection of a sequence of therapeutic agents (and their dosage) to be introduced to the subject. This step of determining the effectiveness is based on a representation (e.g., data storing parameters of a mathematical model) of predicted or expected growth and transition between the plurality of states with introduction of different examples of the therapeutic agents. A treatment strategy is then selected according to the determined effectiveness.

In general, a method for treatment selection includes accepting data characterizing populations of a plurality of cell states at least in part based on a measurement (e.g., tissue sample, bodily fluid sample, or molecular imaging) of a subject's tumor. The plurality of cell states having an overall population, and the cell states including a first cell state (e.g., "a dominant state") with a largest population of the cell states. A specification of each of a plurality of treatment strategies is also accepted. Each treatment strategy represents a selection of a sequence of therapeutic agents to be introduced to the subject. At least some of the treatment strategies are targeted (e.g., associated with reduction in size or reduction in net growth rate) to a cell state other than the first cell state without targeting the first cell state or the overall population of the plurality of cell states. For example, such treatment strategies may not be intuitive or meet conventional therapeutic approaches. An effectiveness of each of the plurality of treatment strategies is determined based on data (e.g., quantitative rates) characterizing expected growth of and transition between the plurality of states with introduction of different agents of the plurality of therapeutic agents, including computing data characterizing evolution of the populations of the plurality of cell states. A treatment strategy is then selected according to the determined effectiveness.

Embodiments may have one or more of the following features.

The data characterizing populations of the plurality of cell states is further based on probabilistic information related to undetected current states or probable future states based on fundamental understanding and/or empirical data regarding cancer evolution.

The data characterizing populations of the plurality of cell states is further based on a measurement of populations of a plurality of cell states in at least one of a tissue sample, a bodily fluid sample, and a molecular image.

Measuring populations of a plurality of cell states includes applying a molecular measurement technique, for instance, a polymerase chain reaction technique.

The determining further includes estimating sizes of each population of the plurality of cell states based on measured population sizes of the plurality of cell states and inferred population sizes of the plurality of cell states.

The data characterizing expected growth of and transition between the plurality of states includes data determined by in vitro and/or in vivo experimentation.

The data characterizing expected growth of and transition between the plurality of states includes information determined by ex vivo experimentation on patient derived biologic material.

Computing data characterizing evolution of the populations includes applying a numerical simulation approach.

The invention is set out in the appended claims, where there is outlined a computer-implemented method for treatment selection using genetic evolutionary dynamics and single cell heterogeneity characterizing a time evolution based on transition rates between different cell types defined by their resistance to therapeutic agents as well as on their individual growth and death rates, the method comprising accepting data characterizing populations of a plurality of heterogenous cell states at least in part based on measurement of a subject's cancer, the plurality of states being defined according to their response to different combinations of therapeutic agents, transition rate from one cell type to another cell type, the plurality of cell states having an overall population, and the cell states including a first cell state having the largest population of the cell states; accepting a specification of each of a plurality of treatment strategies, each treatment strategy representing a selection of a sequence and timing of therapeutic agents to be introduced to the subject, wherein at least some of the treatment strategies are targeted to a cell state other than the first cell state without targeting the first cell state or the overall population of the plurality of cell states; determining using a computer-implemented procedure an effectiveness of each of the plurality of treatment strategies based on data characterizing expected growth of and transition between the plurality of states with introduction of different of the therapeutic agents, including computing data characterizing evolution of the populations of the plurality of cell states, wherein the effectiveness of a treatment strategy is determined from the evolution of the populations of the cell states, by determining the net growth for a cell state, wherein the net growth for the cell of type i is represented by:

$$g_0 x_i + \sum_{j \neq i} T(i,j) g_0 x_j - [\sum_k S_a(i,k) d_k)] x_i$$

assuming that cell death rates are zero in the absence of therapy, that all cells have the same growth rate go, and that the therapeutic agents work by increasing cell death, and wherein $x_i$ represents the cell population of the cell type $i$, $T(i, j)$ represents the transition rate (per cell per generation) from cell type $j$ to $i$; $S_a(i,k)$ represents the sensitivity of cell type $i$ to drug $k$, and $d_k$ represents the normalised dosage of drug $k$; and selecting a treatment strategy according to the determined effectiveness.

There is also provided a treatment selection system using genetic evolutionary dynamics and single cell heterogeneity characterizing a time evolution based on transition rates between different cell types defined by their resistance to therapeutic agents as well as on their individual growth and death rates, the system comprising: an input for accepting data characterizing populations of a plurality of heterogenous cell states at least in part based on measurement of a subject's cancer, the plurality of states being defined according to their response to different combinations of therapeutic agents, transition rate from one cell type to another cell type, the plurality of cell states having an overall population, and the cell states including a first cell state having the largest population of the cell states; a data storage for holding a specification of each of a plurality of treatment strategies, each treatment strategy representing a selected sequence and timing of therapeutic agents to be introduced to the subject, wherein at least some of the treatment strategies are targeted to a cell state other than the first cell state without targeting the first cell state or the overall population of the plurality of cell states; and a computation module configured to determine an effectiveness of each of the plurality of treatment strategies based on data characterizing expected growth of and transition between the plurality of states with introduction of different of the therapeutic agents, including computing data characterizing evolution of the populations of the plurality of cell states, wherein the effectiveness of a treatment strategy is determined from the evolution of the populations of the cell states, by determining the net growth for a cell state, wherein the net growth for the cell of type i is represented by:

$$g_0 x_i + \sum_{j \neq i} T(i,j) g_0 x_j - [\sum_k S_a(i,k) d_k)] x_i$$

assuming that cell death rates are zero in the absence of therapy, that all cells have the same growth rate go, and that the therapeutic agents work by increasing cell death, and wherein $x_i$ represents the cell population of the cell type $i$, $T(i, j)$ represents the transition rate (per cell per generation) from cell type $j$ to $i$; $S_a(i,k)$ represents the sensitivity of cell type $i$ to drug $k$, and $d_k$ represents the normalised dosage of drug $k$; and to select a treatment strategy according to the determined effectiveness.

Embodiments may have one or more of the following advantages.

The approach can identify treatment strategies that may address growth of resistant populations of cells, even if for a period of time such strategies are not optimal to address overall population size. For example, some strategies that are in fact most effective may be counter to current practice that is the standard of care.

The methods described herein can make more effective use of existing drugs, providing greater improvements in survival without having to go to the expense of developing new drugs. For instance, previously unrecognized sequencing of agents may be identified to provide effectiveness that exceeds that achieved with current treatment strategies.

Personalization of treatment strategies or plans is improved by focusing on the details of the distribution of different population states rather than solely on overall or average behavior of a tumor. Different subjects with the same average characteristics of a tumor may in fact have very different optimal strategies if the distribution of various states is different.

In general, conventional personalized medicine treatment regimens take an intuitive approach to treatment of cancer by aiming to reduce the total cell population and/or the population of a dominant cell type of a tumor. In contrast, the system and method described above can result in treatment strategies which counter-intuitively allow the total cell population and/or the population of the dominant cell type of the tumor to increase while causing smaller cell populations within the tumor to decrease. In certain situations, such counterintuitive strategies can result in greater patient longevity.

Other features and advantages of the invention are apparent from the following description, and from the claims.

Description of Drawings

FIG. 1 is a block diagram of a personalized strategic cancer treatment system.

FIG. 2 is a state transition diagram.

FIGS. 3 and 4 are plots of cell populations as a function of time.

Description

1 Overview

Conventional personalized cancer treatment regimens are often designed using the average molecular properties of a sample of a tumor (e.g., obtained by a biopsy) from the subject (e.g., a human subject) and changed only when tumor progression or relapse is detected. However, each tumor cell in a biopsy (which may contain a billion or more tumor cells) can have a unique genetic makeup (i.e., the cell population in a single cancerous tumor can be heterogeneous). Thus, the average molecular properties of a biopsy are not necessarily the best characteristic based on which to select a cancer treatment therapy. As an example of a problem with such an approach, the presence of a relatively small sub-population of resistant cells may ultimately dominate the effectiveness of a therapy, and such an effect may not be evident from average characteristics of the tumor or average response to a sample to therapeutic agents.

Furthermore, cancer is generally genetically unstable as is described by the mutator hypothesis. Conventional personalized cancer treatment regimens are typically based on the most recent sample of a tumor which in many cases is the sample that was used in the initial diagnosis, and the therapy is based on an explicit or implicit assumption that the overall characteristics of the tumor remain static while the size of the tumor is expected to be reduced. However, the properties of a tumor may change from one biopsy to the next, causing the personalized cancer treatment regimen based on the original biopsy to become obsolete and requiring a new therapy to be selected or adjusted.

To cope with these shortcomings of conventional personalized cancer treatment regimens, a strategic personalized cancer treatment regimen described herein employs a mathematical model of the time-evolution of multiple sub-populations to target therapy of a cancerous tumor. Generally, the model accounts for genetic dynamics (such as mutations and chromosomal rearrangements) and single cell heterogeneity, as well as rate of change of population size in the presence of various of the therapeutic agents. In some examples, the model is be used to simulate the behavior (e.g., growth and transition) of the different cell populations in a tumor and to determine a sequence of therapeutic agents that will best achieve a predefined goal, for instance, minimizing the total population size while concurrently minimizing the resistance of certain cell populations to certain treatments.

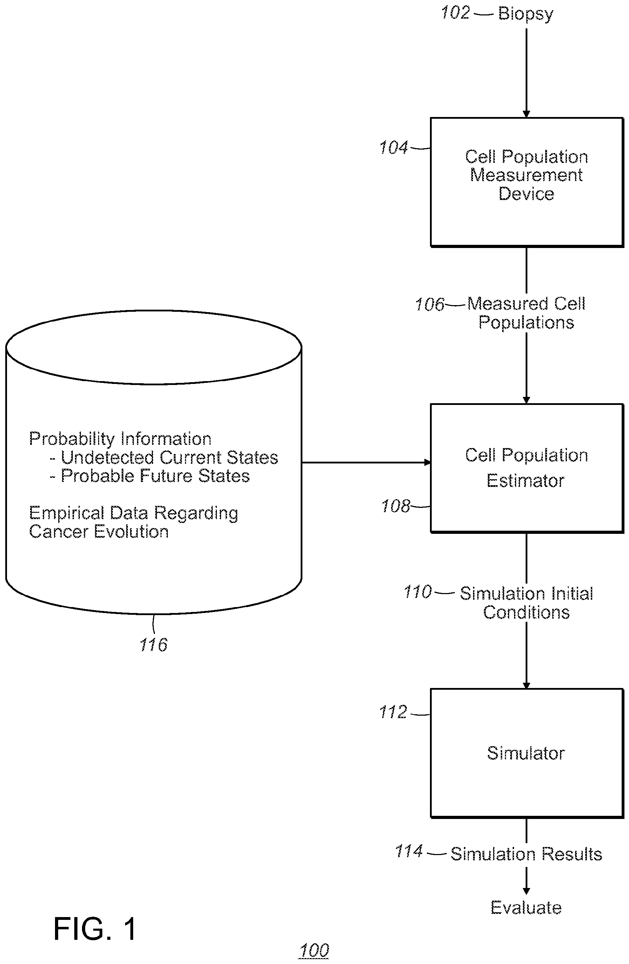

Referring to FIG. 1, a block diagram of a strategic personalized cancer treatment system 100 takes as input a biopsy 102, and generates simulation results 114 which are evaluated by a medical practitioner to select an appropriate treatment strategy.

The biopsy 102 is provided to a cell population measurement device 104 (e.g., a polymerase chain reaction apparatus) which measures the size of each of the cell populations present in the biopsy 102. In general, at the time of this writing, cell population measurement devices 104 are capable of detecting only those populations which are above a certain size threshold. Other populations go undetected. For example, certain cell population measurement devices 104 can detect a cell with a population size of 1 within a total cell population of 10,000.

The measured cell populations 106 output from the cell population measurement device 104 are passed to a cell population estimator 108 which also receives knowledge data 116 which in some examples includes probabilistic information (e.g., the probability of undetected current states or probable future states) and/or empirical data regarding the evolution of cancer cell populations.

Based on the measured cell populations 106 and the knowledge data 116, the cell population estimator 108 generates an estimate of the cell populations in the biopsied tumor. This estimate is an initial condition 110 of the patient's tumor.

The initial condition of the patient's tumor 110 is passed to a simulator 112 which uses a mathematical model to simulate the evolution of the cell populations in the tumor as they are subjected to different combinations of anti-cancer therapies. The simulation results 114 produced by the simulator 112 are output in a form which can be evaluated by a medical practitioner such as an oncologist. The medical practitioner chooses the appropriate treatment strategy based on the simulation results 114.

The output of the system 100 allows an oncologist not only to develop treatment regimens that apply to the current state of the cancerous tumor, but also to develop treatment *strategies* which take into account an estimate of the future course of the cancerous tumor. Such treatment strategies can yield an increased patient survival time. Furthermore, in some examples, counterintuitive treatment strategies which would not have normally been implemented by a medical practitioner are shown to yield greater patient survival time than more conventional treatment regimens.

In some examples, a mathematical optimization, automated control theory, game theoretic, exhaustive enumeration, or other automated approach is used to determine a sequence and timing of application of therapeutic agents based on the mathematical model and a desired goal (e.g., utility function).

Note that although examples may be described in the context of solid tumors and biopsies, other types of cancers, such as hematologic cancers can be addressed using essentially the same approaches (e.g., including sampling of the subject's blood).

## 2 Simple Cell Model

In various examples, different approaches may be used to partition the overall population of cells into subpopulations that are individually modeled. One approach is to individually model different heritable or transient states at a molecular level. Although such an approach may be feasible with sufficient knowledge and/or data, another approach, which is described below, makes use of a set of phenotypical states. In particular, the states are defined according to their response to different combinations of the therapeutic agents. For example, there may be $2^N$ different phenotypical states for a set of N agents.

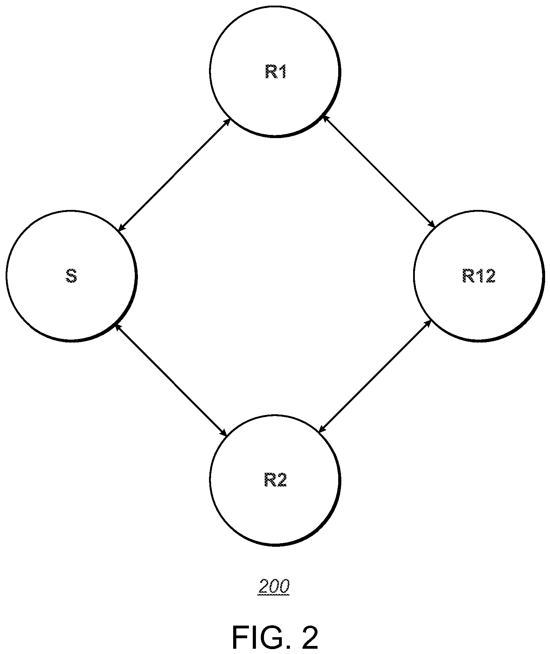

Referring to FIG. 2, a simple model represents a situation in which two therapeutic agents are available, and therefore there are four (i.e., $2^2$) cell sub-populations (i.e., four different phenotypes), which are illustrated as four states in a state diagram 200. The reader should recognize that in many practical situations, the size of the state space would be vastly larger, and potentially have a different type of transition structure. Each state represents a population of cells with different level of sensitivity to two treatments (e.g., anti-cancer drugs or combinations of drugs): $d_1$ and $d_2$. State S is the population of tumor cells that is sensitive (e.g., can be killed or have their growth slowed by) both treatments $d_1$ and $d_2$. State $R_1$ is the population of tumor cells that is sensitive to treatment $d_2$ but is resistant to (e.g., cannot be killed or slowed by) treatment $d_1$. State $R_2$ is the population of tumor cells that is sensitive to treatment $d_1$ but is resistant to treatment $d_2$. State $R_{1\text{-}2}$ is the population of tumor cells that is resistant to both treatments $d_1$ and $d_2$. It is noted that each phenotypic state can be a composite of many thousands of molecular states and transient functional substates, each representative of the same phenotype.

As is illustrated by the arrows in the state diagram, as a patient is treated with anti-cancer drugs, the population of cells of a state can change (i.e., grow or decline) from transition from one state to another state as illustrated. For example, a cell in the population at state S may mutate and become resistant to treatment $d_1$ over the course of time, thereby causing the cell to transition to the population at state $R_1$. Furthermore, cell growth and cell death in each population (and the total population as a whole) is affected by the drugs in a dose dependent manner. In some examples, partial resistance can occur.

## 3 Mathematical Model of the System

One approach to predicting the population growth rate of each cancerous cell type can be modeled by a differential equation as is described below. To simplify the following description as introduced above, it is assumed that there are two "drugs" (understanding that references to "drugs" can equivalently be replaced with combinations of drugs, or other forms of treatment) available for treating cancer, $d_1$ and $d_2$, and any combination of their dosage can be chosen for treatment. However, the total dose of both drugs is limited by toxicity; generally, one cannot give as much of the drugs in combination as individually. In the example below, the sum of the two doses cannot exceed a normalized dose of 1. As is illustrated in FIG. 2, a cancerous tumor being treated by two drugs can be seen as including four disjoint tumor cell types.

- Cell type S is sensitive to both drugs.

- Cell type $R_1$ is resistant to drug 1 and sensitive to drug 2.

- Cell type $R_2$ is resistant to drug 2 and sensitive to drug 1.

- Cell type $R_{1\text{-}2}$ is resistant to both drugs.

In some examples, the attributes "sensitive" and "resistant" are quantitatively defined by the ratio of the cell death rate induced by treatment and the natural growth rate. It is assumed that the population growth rate of each cell type depends on (1) the natural growth rate, (2) the cell death rate caused by a treatment (i.e., according to the selected agent(s) that are present at time t), and (3) influx into the population due to mutation rates from other closely related cell types.

We denote a four-component vector, $\vec{x} = (x_S, x_{R1}, x_{R2}, x_{R1-2})$, as the cell population of each class. We assume that cell death rates are zero in the absence of therapy, that all cells have the same growth rate, and that the drugs work by increasing cell death. For each class, $i \in \{S, R_1, R_2, R_{1-2}\}$, the net growth rate is

$$g_0 x_i + \sum_{j \neq i} T(i,j) g_0 x_j - (S_a(i,1)d_1 + S_a(i,2)d_2)x_i :$$

The first term corresponds to the growth rate of cell type i with a rate $g_0$ shared by all cell types. The second term corresponds to the transitions from all other cell types, where $T(i,j)$ specifies the transition rate (per cell per generation) from cell type $j$ to i. We assume that (i) transition rates from resistant to sensitive cell types are negligible, (ii) the transition rate of acquiring the resistance to one drug is independent of the resistance phenotype to another drug, and (iii) transition rates of acquiring double resistance in one step are negligible. Thus, $T(R_1,S) = T(R_{1-2},R_2)$, $T(R_2,S) = T(R_{1-2}, R_1)$, and all other entries of $T$ are zero. The third term corresponds to the treatment-caused cell death, where $(d_1,d_2)^T \equiv \vec{d}$ represents the normalized dosages of the two drugs ($d_1$ is drug-1 and $d_2$ is drug-2) with the constraints $0 \leq d_1; d_2, d_1 + d_2 \leq 1$ and $S_a(i,1)$, $S_a(i,2)$ represents the sensitivities of cell type i to drug-1 and drug-2. The population dynamics of the four cell types can be compactly expressed as a matrix differential equation:

$$\frac{d\vec{x}}{dt} = \left[ (I+T)G_0 - \text{diag}(S_a\vec{d}) \right] U(\vec{x}-1)\vec{x} \tag{1}$$

where $I$ denotes a four-by-four identity matrix and diag(.) denotes an operator of placing vector components on the diagonal entries of a zero matrix. $U(\vec{x} - 1)\vec{x}$ sets component values to 0 if they are less than 1; that is, $U(\vec{x} - 1) = 0$ for x < 1 and $U(\vec{x} - 1) = 1$ for $x \geq 1$. This term stipulates that fractional cell numbers (less than 1 cell) do not contribute to cell division.

The above equation can be solved analytically if $\vec{d}(t)$ is piecewise constant. This is the case for practical treatments, because the dosages are altered only at fixed time intervals or when the current treatment regimen fails. Suppose with time interval $[0, T]$ $\vec{d}(t) \equiv \vec{d}$ is a constant. If all components of $\vec{x} > 1$ then the above equation is then reduced to a first-order linear matrix differential equations

$$\frac{d\vec{x}}{dt} = \left[ (I+T)G_0 - \text{diag}(S_a\vec{d}) \right] \vec{x} \equiv A\vec{x} . \tag{2}$$

The solution of this equation is simply the matrix exponential times of the initial population $\exp(At)\vec{x}(0)$. This solution sustains as long as $\vec{d}(t)$ stays constant and all components of $\vec{x} > 1$.

If some components of $\vec{x} < 1$, (e.g., $x_{R1-2} < 1$), this second equation is no longer equivalent to the first equation. $U(\vec{x} - 1)\vec{x}$ sets fractional cell numbers to 0, and thus blocks their contribution to the growth rates. In this case, we define a matrix B such that the rows corresponding to the fractional populations are 0 and the remaining rows are equal to those of A . Hence, the first equation becomes

$$\frac{d\vec{x}}{dt} = B\vec{x} \tag{3}$$

and the solution is $\exp(Bt)\vec{x}(0)$. This third equation is valid only if the population of each cell type does not cross the boundary of one cell (increases from x < 1 to x > 1 and vice versa). When boundary crossing occurs, the constant matrix B has to be updated, and the population before boundary crossing is treated as the initial population for the new differential equation.

Prediction of each population is summarized as follows:

1. Divide time into regular intervals (45 days in this application). $\vec{d}(t)$ are constants within each interval but are allowed to vary between intervals.

2. At the beginning of each interval, update equation 1 by replacing $\vec{d}(t)$ with the new dosage combination and setting the initial population as the calculated population of the prior interval.

3. Construct the constant matrix B from $[(I + T)g_0 - \text{diag}(S_a\vec{d})]U(\vec{x} - 1) \equiv A$ and the initial population of the current interval. Set the rows of B corresponding to fractional populations to 0.

4. Denote $\vec{x}(T_i)$ the initial population of the interval $[T_i, T_{i+1})$, and $B_t$ the corresponding constant matrix in equation 3. The solution for $t \in [T_i, T_{i+1})$ is $\vec{x}(t) = \exp(B_i(t - T_i))\vec{x}(T_i)$ if entries of $\vec{x}(t)$ do not cross the single cell boundary.

5. If boundary crossing occurs on $\tau \in [T_i, T_{i+1}]$, then update $B_i$ accordingly and set the initial population of equation 3 to $\vec{x}(\tau)$.

6. Continue matrix and initial population updates to the end of the total time considered (5 years in this study).

In order to predict the evolution of the populations, an initial condition is used. Generally, this initial condition is based on results of a biopsy of the subject. In some examples, the setting of the initial condition does not necessary equal the populations observed in a tissue sample, for example, to account for the possible non-zero but sub-threshold of detection populations in certain states. In some examples, specific characteristics of the subject's tumor (e.g., genetic traits), together with empirical or basic knowledge of evolutionary courses of similar tumors, may be used in the determination of the parameters and/or initial conditions of the model.

Note that in this example, a deterministic model is used to predict the evolution of the populations of the different states. In other examples, random components may be introduced into the model, for example, through uncertainty in the model parameters (e.g., the growth, death, and transition rates) and/or through modeling of the random perturbation ("driving noise") in the predicted derivatives. Similarly, uncertainty in initial conditions may be models as well to account for the imperfect knowledge that is gained from a sample from the subject.

In some examples, the approaches make use of local or centralized databases or centralized computing resources providing parameters of the model. In some examples, the mapping from tissue sample to model parameters or initial condition makes use of such centralized resources.

4 Treatment Selection

By using the mathematical model that can combine knowledge gained by an initial (as well as subsequent) tissue samples and the knowledge of the predicted evolution of the populations, various approaches to treatment selections may be used. One example of a characterization of a treatment is as a sequence of fixed duration intervals during each of which a particular therapeutic agent is introduced at a constant dosage (or possibly no new agent), with the agent(s) and dose(s) being changed in the instant between these intervals.

A treatment strategy includes a method for selecting a treatment. One approach is to have a set of pre-established strategies, and based on the model, the best strategy is selected. This approach is motivated by, and in some implementations makes use of mathematical techniques in the field of game theory. Other approaches may be based on optimization techniques and/or optimal control techniques that account for uncertainty in the models and initial estimates.

4.1 Selection from a Strategy Set

In some examples, heuristic goals are used to design and simulate a treatment strategy. Some examples of heuristic strategies are:

- Choose a sequence of drug combinations that minimizes a predicted total population of cells in a tumor.

- Choose a sequence of drug combinations that minimizes the risk of incurable cells developing unless there is an immediate risk of mortality.

- Minimize the risk of the predicted total population of cell in a tumor unless the model predicts the appearance of the first incurable cell at the next interval, in which case minimize the probability of appearance of an incurable cell.

- Estimate the time to either incurability or death and react to the most proximal threat, as long as there is a chance of cure

Generally, each of a set of predefined strategies, which can include conventional strategies, are evaluated with the model, and the best strategy is selected. Based on this approach, we can demonstrate the advantages of strategy-based treatments (adjusting drug dosages according to the predicted risks) over the current paradigm of personalized medicine (choosing treatments based on the molecular properties of the predominant sub-population, and change drugs when tumor progression or relapse is detected). (We note that more complex individualized strategies such as the ones below are also personalized medicine, just not as currently practiced). To fulfill this goal we implemented 6 treatment strategies in simulation studies. We assume in this simulation that the cell type S is more sensitive to drug 1 (di) than to drug 2 ($d_2$), and that $R_2$ is more sensitive to $d_1$ than $R_1$ is to $d_2$. Hence, $d_1$ is overall the superior drug.

Strategy 0: Current personalized medicine strategy. Initially treat the patient with $d_1$ alone if

$$\frac{x_{R1}}{x_S + x_{R1} + x_{R2} + x_{R12}} \leq 0.5$$

(i.e., the $R_1$ population does not dominate the tumor). Otherwise treat the patient with $d_2$ alone. A nadir is a local minimum of the total population among the time-series profile where the current treatment is maintained. Maintain the current treatment until either one of the following events occur: (a)The total population reaches twice the nadir population (RECIST tumor progression scaled up to represent tumor volume rather than a single linear dimension), or (b)The total population reemerges from a level below the detection threshold ($10^9$) (relapse). If either (a) or (b) occurs then switch to another drug. Strategy 0 mimics the current paradigm of personalized medicine, in that the initial treatment is selected by molecular characterization of the predominant population, and classification into one of the 4 cell types.

Strategy 1: Minimize the predicted total population. Every 45 days, adjust $\vec{d}(t)$ to minimize the predicted total population by maintaining the (hypothetical) treatment over a period of "lookahead time". Vary $d_1$ and $d_2$ between 0 and 1 with a 0.01 interval. For each dosage combination, evaluate the predicted total population by solving equation 1 with the initial populations being the currently observed populations of each cell type, $\vec{d}(t)$ being fixed to the given dosage combination, and the duration being the lookahead time (either 45 or 60 days). Choose the dosage combination that minimizes the predicted total population.

Strategy 2: Minimize the risk of incurable cells developing unless there is an immediate threat of mortality. Every 45 days, adjust $\vec{d}(t)$ to minimize the predicted $R_{1-2}$ population if the total population does not exceed a threshold. $R_{1-2}$ is resistant to both drugs and therefore it is often incurable. All simulations start with an $R_{1-2}$ population of 0. By preventing the formation of $R_{1-2}$, the possibility for long term disease control and/or cure is maintained. If the total population exceeds the threshold then adjust $\vec{d}(t)$ to minimize the predicted total population. We implement two threshold values in simulation studies: strategy 2.1:$10^9$, strategy 2.2:$10^{11}$. Strategy 2 places prevention of the double resistant mutant $R_{1-2}$ with a higher priority than reduction of the total population, unless the total population has reached a threshold to threaten the patient's life. The rationale is that suppressing $R_{1-2}$ can possibly cure the patients.

Strategy 3: Minimize the predicted total population unless there is a prediction that the first incurable cell will form within 45 days. Every 45 days, adjust $\vec{d}(t)$ to minimize the predicted total population if the predicted $R_{1-2}$ population < 1. Otherwise adjust $\vec{d}(t)$ to minimize the predicted $R_{1-2}$ population. However, if the current $x_{R12} \geq 1$ and $R_{1-2}$ is not curable ($g_0 - S_a(R_{1-2},1) - S_a(R_{1-2},2) > 1$), then minimize the predicted total population. The rationale is to switch to prevention of $R_{1-2}$ only if the predicted risk of $R_{1-2}$ emergence is prominent. If $R_{1-2}$ has already appeared, then minimize the total population without being concerned with $R_{1-2}$ prevention. Given that we allow for "relative" resistance, it is possible that $R_{1-2}$ is not incurable, but in most of our parameter settings it is incurable.

Strategy 4: Estimate the time to either incurability or death and react to the most proximal threat, as long as there is a chance of cure. Every 45 days, evaluate the predicted durations toward incurability ($x_{R1-2} \geq 1$) and mortality (population $\geq 10^{13}$) dictated by the growth of $S$, $R_1$, $R_2$ and $R_{1-2}$ populations. For each dosage combination $\vec{d}$, define $\tau_{inc}(\vec{d})$ the predicted duration of reaching incurability ($x_{R1-2} \geq 1$) conditioned on the initial population as the currently observed population and the drug dosage fixed to $\vec{d}$. Define $\tau_S(\vec{d})$ the predicted duration of $x_S$ reaching mortality ($x_S \geq 10^{13}$) conditioned on the initial population as the currently observed population and the drug dosage fixed to $\vec{d}$. $\tau_{R1}$, $\tau_{R2}$, $\tau_{R1-2}$ can be defined in the same fashion. If the current $x_{R12} < 1$ or $R_{12}$ is curable (there exists some $\vec{d}$ such that each component of diag($S_a\vec{d}$) > $g_0$), then vary $\vec{d}$ to maximize min($\tau_{inc}$, $\tau_S$, $\tau_{R1}$, $\tau_{R2}$, $\tau_{R1-2}$) with the constraint that min($\tau_S$, $\tau_{R1}$, $\tau_{R2}$, $\tau_{R12}$) > 45 days. If such dosage combination does not exist, then maximize min($\tau_S$, $\tau_{R1}$, $\tau_{R2}$, $\tau_{R1-2}$). If the current $x_{R1-2} \geq 1$ and $R_{1-2}$ not curable, then maximize min($\tau_S$, $\tau_{R1}$, $\tau_{R2}$, $\tau_{R1-2}$). The rationale is to quantify the risk induced by each cell type as the predicted durations toward incurability or mortality, and find the dosage combination to minimize the risk.

### 4.2 Other approaches

In other examples, mathematical optimization techniques can be used to determine the best (e.g., globally optimal over a finite or infinite horizon) sequence of drug combinations. An example of a utility function is a patient's survival time. In some examples, constraints on possible treatments are also introduced, for example, to account for toxicity of the agents, thereby avoiding strategies or dosages that would harm the subject. Without necessarily considering the computational complexity of the approach, at least conceptually, all treatments (i.e., sequences of agents, dosages from a finite or continuous set of levels) can be enumerated to determine a global optimum with respect to a utility measure, which in general depends on both an overall population, and populations of particular states. Mathematical techniques from the fields of optimization and/or optimal control can be applied to reduce the computation required, for instance, in some cases achieving close to optimal treatments.

In some examples, techniques such as game theory may be employed for quantitative evaluation and selection of strategic choices.

### 5 Examples

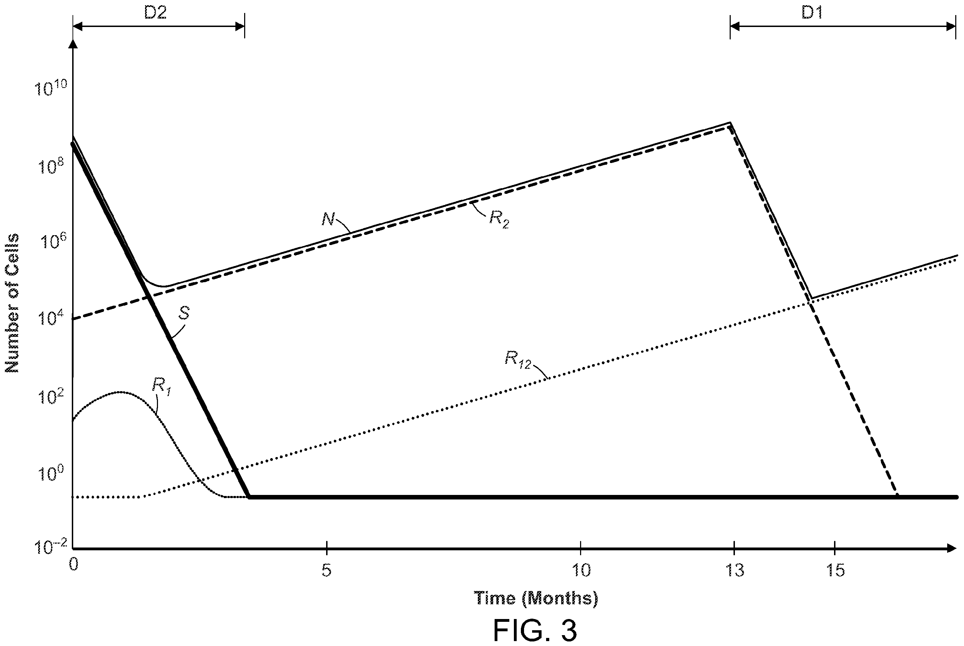

Referring to FIG. 3, a graph illustrates an example the progression of cancer cell population sizes over the course of a conventional personalized medicine treatment regimen, the regimen including two drugs $d_1$ and $d_2$. The graph plots a number of cell population sizes vs. time, including plot lines for the total cell population size, $N$, as well as plot lines for the sub populations $S$, $R_1$, $R_2$, $R_{1-2}$ .

In this example of a conventional personalized medicine treatment, a patient visits a personalized medicine oncologist due to the discovery of a 1 $cm^3$ cancerous mass. The oncologist biopsies the mass and the biopsy indicates that the mass includes only cells of type S (i.e., cells that are sensitive to both treatments $d_1$ and $d_2$). In some examples, the types of cells included in the biopsy are determined using a molecular test, for instance without limitation, using polymerase chain reaction (PCR) technology. At the time of this writing such a molecular test is ideally capable of finding 1 non-S type cell in 10,000 total cells. In some examples, less sensitive molecular tests are used.

For purposes of this example, it is assumed that drug $d_2$ is capable of efficiently killing S type cells and can completely eradicate a detectable tumor which includes mostly S cells. It is also assumed that drug $d_1$ is less effective at treating S type cells, only slowing their growth.

Given the above assumptions and the information from the biopsy, the oncologist identifies $d_2$ as the best drug for treating the patient's tumor. As can be seen from the S cell type curve, the patient is treated with drug $d_2$ and the population of S type cells is drastically reduced within five months. Furthermore, the overall tumor size, N, is reduced to the point where it can no longer be detected using a CAT scan.

However, in this example the information provided by the biopsy was inaccurate and in reality the tumor actually included 100,000 cells of the $R_2$ type (i.e., those which are resistant to the $d_2$ drug). The inaccuracy was due to the fact that the $R_2$ cells exist below the minimal threshold of detection of the test. In this example, it is assumed that the $R_2$ type cells are genetically closer to the $R_{1-2}$ type cells than the $R_1$ type cells. Thus, the $R_2$ type cells can more quickly transition and become $R_{1-2}$ cells (e.g., by heritable transitions such as single nucleotide changes or chromosomal rearrangements or non-heritable mutations such as epigenetic mutations).

At the 13 month point, the patient experiences a detectable relapse. A biopsy indicates that the tumor includes $R_2$ type cells which are most effectively treated with drug $d_1$. The patient's tumor is treated with $d_1$ and the overall size of the tumor, N, reduces as the $R_2$ cell population is eradicated.

However, due to the fact that the $R_2$ type cells were not treated until the 13 month point, some of the population of $R_2$ cells mutated to $R_{1-2}$ cells (i.e., cells which are not treatable by either of the drugs $d_1$ or $d_2$) at roughly the two month point. The cells of type $R_{1-2}$ continue to grow until at 26 months another relapse occurs, at which time the tumor is untreatable and thus incurable.

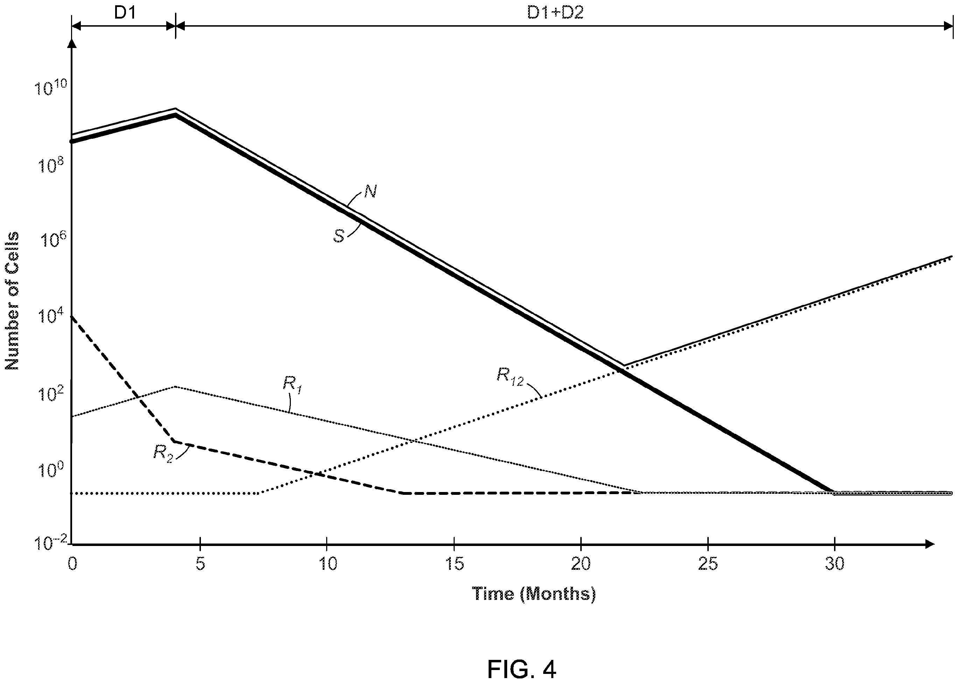

Referring to FIG. 4, another graph illustrates an example the progression of cancer cell population sizes over the course of a personalized *strategic* medicine treatment regimen, the regimen utilizing the same two drugs that were used in the previous example: $d_1$ and $d_2$. The graph plots a number of cell population sizes vs. time, including plot lines for the total cell population size, N, as well as plot lines for the sub populations S, $R_1$, $R_2$, $R_{1-2}$ .

In this example of a conventional strategic personalized medicine treatment, the patient from the previous example visits a personalized strategic medicine oncologist due to the discovery of a 1 $cm^3$ cancerous mass. The oncologist biopsies the mass and the biopsy indicates that the mass includes only cells of type S (i.e., cells that are sensitive to both treatments $d_1$ and $d_2$). However, based on the collective knowledge represented by epidemiologic and molecular oncology data accumulating in the national and international databases, the oncologist recognizes that even though no $R_2$ type cells were detected, there is a chance that this type of tumor might include a small minority subclone of $R_2$ cells. Furthermore, the oncologist recognizes that $R_2$ type cells present a high risk of mutating to cells of type $R_{1-2}$, which are incurable.

As was the case in the previous example, it is assumed that drug $d_2$ is capable of efficiently killing S type cells and can completely eradicate a detectable tumor which includes mostly S cells. It is also assumed that drug $d_1$ is less effective at treating S type cells, only slowing their growth.

Based on this information, the oncologist strategically treats the patient with drug $d_1$ for four months, minimizing the population of $R_2$ type cells. Since $d_1$ is less effective than $d_2$ at killing cells of type S, the total population of cells, N, slowly grows during the initial treatment with $d_1$. After four months of treating with $d_1$, the oncologist determines that allowing the total population of cells, N, to continue to grow poses unacceptable immediate risks, and then switches to a mixture of $d_1$ and $d_2$. After the point of treatment with the mixture of $d_1$ and $d_2$ the tumor eventually disappears below the level of detection. In this example, a population of $R_{1-2}$ cells doesn't appear until roughly the eight month point.

Due to the treatment strategy described above, the patient does not present with a incurable relapse of $R_{1-2}$ cells until 38 months from the date of initial treatment, causing the patient to live an extra year.

6 Linkage to Other Models

The current model focuses on drug sensitivity/resistance phenotypes as determined by genetic and epigenetic factors and their influence on optimal personalized medicine factors. The focus on heritable phenotypes and the condensation of a very large number of genotypes onto a smaller number of phenotypic clusters are both essential to allow computationally feasible evaluation of complex treatment strategies. We term this focused model the "core model." Supported by extensive sensitivity analysis over a broad range of parameters, the core model has produced high-level conclusions about personalized medicine strategies.

In applying this core model in detail to real tumors, many additional complexities will need to be taken into account. These additional complexities will need to be evaluated by separate linked models, which then feed information to the core model. This framework of linked models then allows representation of the complexities of individual tumors and therapies so that detailed conclusions can be drawn. This is a more efficient approach for a complex system in that properties of individual tumors can be computed in separate steps and then applied to a large number of candidate treatment strategies.

When applied to real tumors, the core model will, in many cases, need to specify probability distributions of parameter values rather than discrete values. The current core model uses broad ranges of parameter values with a uniform distribution (each value of the parameter is assigned equal probability); however, when linked to other sources of information, these probability distributions may be narrowed down and become more structured. Below, we give a high-level concept of how the core model would assimilate information from other models about (i) the heritable states underlying a sensitivity or resistance phenotype and transitions between these states, (ii) passenger vs. driver mutations, (iii) nongenetic mechanisms of resistance, and (iv) biodistribution.

The following sources would be used to get a mapping between phenotypic sensitivity/resistance states and underlying genotypes, as well as to estimate the probability of existence of a particular state:

i) Direct measurement: This requires the development of noninvasive methods for sampling and molecular and/or phenotypic characterization at the single-cell level.

ii) Empirical databases: By collecting information on a large number of patients at diagnosis and autopsy, one can begin to characterize the possible states and their likelihood of occurrence empirically. For example, in a recent paper, the detailed subclonal structure of approximately 100 triple-negative breast cancers was presented, albeit not yet at single-cell resolution. Molecular studies of panels of cell lines can be used to supplement this empirical information, and these cell lines can be directly tested for drug sensitivity phenotypes to correlate with the genetic and epigenetic annotations.

iii) Computational pathway analysis: As an example PARADIGM software (University of California at Santa Cruz Cancer Genomics Browser) can analyze over 1,400 curated signaling pathways. Such software may be used in the future in an attempt to predict what molecular substates may be associated with a particular sensitivity profile in that a variety of specific modifications might present with similar phenotypic effects analyzed at the pathway level. By using this software, it might be possible to predict additional genetic states associated with a phenotype based on their pathway relationship to others that had been determined experimentally.

iv) Functional genomics: High-throughput screens with siRNA and shRNA can be applied systematically to predict what genotypes might be associated with sensitivity and resistance phenotypes.

The transitions between these states may be by any mechanism, not limited to mutations of a single driver gene but including all known mechanisms of genetic and epigenetic change (mutation, insertion, deletion, translocation, amplification, chromosome loss or gain, DNA methylation, or histone modification). The total transition rate between phenotypic states A and B is the sum of the rates corresponding to all possible ways of getting from phenotype A to phenotype B. Given that we may not know which of many underlying molecular states is currently resulting in phenotype A, we may have to calculate the rate of transition to B for each possible molecular state underlying phenotype A and add up all these rates, each multiplied by the probability of a particular molecular state underlying the phenotype. For any individual rate, we will need to know how similar the two molecular states are and the rates of possible interconversion mechanisms. Because genetic instability mutations may affect these individual rates, the individual rates themselves may need to be represented as ranges or probability distributions.

The model may be considered to fundamentally be a model of phenotypic transitions between drug sensitivity states, and the phenotypic transition may occur via any of a large number of possible genetic changes. The total rate of phenotypic change is the sum of the rates from all the individual changes that could, in principle, lead to the phenotypic change. For drug resistance, this could be by acquisition of a new driver mutation that circumvents the previous therapy, but it could also be due to a passenger mutation. Such a mutation, although not implicated in driving the tumor originally, may drive resistance. For example, a mutation may occur that leads to alterations in cellular distribution or metabolism of the drug. We have previously written about the fact that passenger mutations represent a reservoir of diversity that may also contribute to drug resistance, and therefore survival under drug therapy.

Passenger mutations differ from driver mutations in that they are not selected for in the absence of therapy. This is reflected in the net growth rate parameters in the absence of drug therapy in the model, which will not reflect an increase in growth rate with the acquisition of a passenger mutation.

Non-genetic resistance mechanisms: Known mechanisms of resistance to vemurafenib exemplify resistance mechanisms that do not involve genetic change. In particular, in colorectal cancer, vemurafenib inhibition of B-Raf leads to feedback up-regulation of EGF receptor (EGFR), in turn, leading to two events: (i) upstream activation of Ras, leading to dimerization of B-Raf, rendering vemurafenib ineffective and (ii) parallel activation of the pI3-kinase signaling pathway, potentially circumventing the Ras-Raf-Mek pathway to the extent that it may still be inhibited. This resistance is hard-wired, occurs rapidly, and does not require genetic change. Such feedback loops are common in signaling pathways, and, in fact, a similar feedback loop affects pI3-kinase pathway inhibitors.

As noted above, "drug-1" and "drug-2" may also refer to combinations directed at single states. This means, for instance, in a case such as vemurafenib in colorectal cancer, vemurafenib clearly should be given in combination with an EGFR inhibitor. If the heritable state is that described for colorectal cancer, drug-1 means an optimized drug or drug combination for the transient adaptations that can be assumed within that heritable state (i.e., a combination, such as vemurafenib and cetuximab). The optimized combination must be determined by a linked model separate from the core model. High-content phosphoproteomics is an important source of information in attempting to understand signaling as it relates to nongenetic mechanisms of resistance.

In some cases, an optimized combination to deal with nongenetic resistance is not available. In this case, that fact is reflected in lesser net efficacy parameter input into the model. In other cases, nongenetic resistance may be variable. In this case, it can be represented by a probability distribution of the efficacy parameter. This probability distribution could be different in different genetic states; that is, genetic states could influence the likelihood of a particular resistance mechanism. All this can be input into the parameter distributions in the model if it is known.

Biodistribution: Just as drug-1 and drug-2 are optimized combinations if necessary to deal with nongenetic resistance mechanisms, the dose and schedule of drug-1 or drug-2 given as a single agent are assumed to be optimized with respect to drug delivery. To the extent that the continuum of intratumoral concentrations corresponding to a dose is known, this information can be fed into the core model's efficacy parameter distribution. We are actively researching the problem of determining the optimal dose for antibodies as a function of their biodistribution and the biophysical factors that determine this. These problems may be currently unsolved, but information can be fed from complex models of this phenomenon into the core model if available.

7 Alternatives

While the description above describes a simple two drug, four state model, more complex models can be implemented. For example, a typical model may include up to 1000 experimental and approved anti-cancer drugs. In such cases, at least conceptually the size of the state space may be very large (e.g., $2^{1000}$). The size of the state space may be reduced by various techniques, for example, pre-selection of a subset of the agents, or modeling of classes of the agents. Even with a large number of alternative agents, the approaches above can be used to compare particular treatments, and in optimization approaches can be used to iteratively search for a best treatment, for example, by an iterative refinement approach.

In some examples, other models of cancerous tumor growth such as the model described by Norton-Simon can be used.

In the above description, personalized strategic cancer treatment is described in the context of treating a human subject. It should be known that any other organism that experiences unregulated cell growth (e.g., mammals such as dogs, reptiles, etc.) can be the subject of the approaches described above.

In some examples, ex vivo experimentation is performed on patient derived biological material (e.g., a tumor tissue sample) to determine a representation of expected growth and transition between a plurality of cell states. In other examples, in vitro or in vivo experimentation is performed on a tumor a tissue sample, cell lines, or other biological material, to determine a representation of expected growth and transition between a plurality of cell states.

8 Implementations

The techniques and all of the functional operations described in this specification can be implemented in digital electronic circuitry, or in computer hardware, firmware, software, or in combinations of them. The system can be implemented as a computer program product, i.e., a computer program tangibly embodied in an information carrier, e.g., in a machine-readable storage device or in a propagated signal, for execution by, or to control the operation of, data processing apparatus, e.g., a programmable processor, a computer, or multiple computers. A computer program can be written in any form of programming language, including compiled or interpreted languages, and it can be deployed in any form, including as a stand-alone program or as a module, component, subroutine, or other unit suitable for use in a computing environment. A computer program can be deployed to be executed on one computer or on multiple computers at one site or distributed across multiple sites and interconnected by a communication network.

In some examples, the implementation can be at least partially centralized such that information regarding a subject tissue sample is provided to a central computing resource (e.g., a remote computer) and a treatment is provided in return. In some implementations, the processing of the sample is automated and/or results of analysis of a sample are automatically provided such a computing resource, and the treatment (e.g., agents and dosing) are provided to a clinician in return.

Method steps of the system can be performed by one or more programmable processors executing a computer program to perform functions of the system by operating on input data and generating output. Method steps can also be performed by, and apparatus of the system can be implemented as, special purpose logic circuitry.

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for executing instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. Information carriers suitable for embodying computer program instructions and data include all forms of non-volatile memory, including by way of example semiconductor memory devices, e.g., EPROM, EEPROM, and flash memory devices; magnetic disks, e.g., internal hard disks or removable disks; magneto-optical disks; and CD-ROM and DVD-ROM disks. The processor and the memory can be supplemented by, or incorporated in special purpose logic circuitry.

To provide for interaction with a user, the system can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. Interaction with a user does not need to be direct. The system can be implemented with an application programming interface allowing alternative means of exchanging input data and output data with the system.

Further technical details, examples, embodiments, and/or experimental results are found in "Impact of genetic dynamics and single-cell heterogeneity on development of nonstandard personalized medicine strategies for cancer," Robert A. Beckman, et al., Proc. National Academy of Science of the U.S.A. 2012 Sep. 4; 109(36): 14586-91, including the Supplemental Information for the article available to the public from the National Academy of Science (e.g., via http://www.pnas.org/content/109/36/14586/suppl/DCSupplemental).

It is to be understood that the foregoing description is intended to illustrate and not to limit the scope of the invention, which is defined by the scope of the appended claims.

**Claims**

1. A computer-implemented method for treatment selection using genetic evolutionary dynamics and single cell heterogeneity characterizing a time evolution based on transition rates between different cell types defined by their resistance to therapeutic agents as well as on their individual growth and death rates, the method comprising:

accepting data characterizing populations of a plurality of heterogenous cell states at least in part based on measurement of a subject's cancer, the plurality of states being defined according to their response to different combinations of therapeutic agents, transition rate from one cell type to another cell type, the plurality of cell states having an overall population, and the cell states including a first cell state having the largest population of the cell states;

accepting a specification of each of a plurality of treatment strategies, each treatment strategy representing a selection of a sequence and timing of therapeutic agents to be introduced to the subject, wherein at least some of the treatment strategies are targeted to a cell state other than the first cell state without targeting the first cell state or the overall population of the plurality of cell states;

determining using a computer-implemented procedure an effectiveness of each of the plurality of treatment strategies based on data characterizing expected growth of and transition between the plurality of states with introduction of different of the therapeutic agents, including computing data characterizing evolution of the populations of the plurality of cell states, wherein the effectiveness of a treatment strategy is determined from the evolution of the populations of the cell states, by determining the net growth for a cell state, wherein the net growth for the cell of type i is represented by:

$$g_0 x_i + \sum_{j \neq i} T(i,j) g_0 x_j - [\sum_k S_a(i,k) d_k)] x_i$$

assuming that cell death rates are zero in the absence of therapy, that all cells have the same growth rate go, and that the therapeutic agents work by increasing cell death, and wherein $x_i$ represents the cell population of the cell type $i$, $T(i,j)$ represents the transition rate (per cell per generation) from cell type $j$ to $i$; $S_a(i,k)$ represents the sensitivity of cell type i to drug $k$, and $d_k$ represents the normalised dosage of drug $k$; and

selecting a treatment strategy according to the determined effectiveness.

2. The method of claim 1 wherein the data characterizing populations of the plurality of cell states is further based on knowledge data (116) including probabilistic information including undetected current states or probable future states.

3. The method of claim 1 wherein the data characterizing populations of the plurality of cell states is further based on a measurement of populations of a plurality of cell states in at least one of a tissue sample, a bodily fluid sample, and a molecular image.

4. The method of claim 3 wherein measuring populations of a plurality of cell states includes applying a molecular measurement technique.

5. The method of claim 4 wherein applying the molecular measurement technique includes applying a polymerase chain reaction technique.

6. The method of claim 1 wherein the determining further includes estimating sizes of each population of the plurality of cell states based on measured population sizes of the plurality of cell states and inferred population sizes of the plurality of cell states.

7. The method of claim 1 wherein the data characterizing expected growth of and transition between the plurality of states includes data determined by in vitro and/or in vivo experimentation.

8. The method of claim 1 wherein the data characterizing expected growth of and transition between the plurality of states includes information determined by ex vivo experimentation on patient derived biologic material.

9. The method of claim 1 wherein each treatment strategy represents a selection of a sequence, timing, and dosage of therapeutic agents to be introduced to the subject.

10. A treatment selection system using genetic evolutionary dynamics and single cell heterogeneity characterizing a time evolution based on transition rates between different cell types defined by their resistance to therapeutic agents as well as on their individual growth and death rates, the system comprising:

an input for accepting data characterizing populations of a plurality of heterogenous cell states at least in part based on measurement of a subject's cancer, the plurality of states being defined according to their response to different combinations of therapeutic agents, transition rate from one cell type to another cell type, the plurality of cell states having an overall population, and the cell states including a first cell state having the largest population of the cell states;

a data storage for holding a specification of each of a plurality of treatment strategies, each treatment strategy representing a selected sequence and timing of therapeutic agents to be introduced to the subject, wherein at least some of the treatment strategies are targeted to a cell state other than the first cell state without targeting the first cell state or the overall population of the plurality of cell states; and

a computation module configured to determine an effectiveness of each of the plurality of treatment strategies based on data characterizing expected growth of and transition between the plurality of states with introduction of different of the therapeutic agents, including computing data characterizing evolution of the populations of the plurality of cell states, wherein the effectiveness of a treatment strategy is determined from the evolution of the populations of the cell states, by determining the net growth for a cell state, wherein the net growth for the cell of type i is represented by:

$$g_0 x_i + \sum_{j \neq i} T(i,j) g_0 x_j - [\sum_k S_a(i,k) d_k)] x_i$$

assuming that cell death rates are zero in the absence of therapy, that all cells have the same growth rate go, and that the therapeutic agents work by increasing cell death, and wherein $x_i$ represents the cell population of the cell type $i$, $T(i, j)$ represents the transition rate (per cell per generation) from cell $type\,j$ to $i$; $S_a(i,k)$ represents the sensitivity of cell type i to drug $k$, and $d_k$ represents the normalised dosage of drug $k$; and

to select a treatment strategy according to the determined effectiveness.

11. The treatment selection system of claim 10 further comprising a database accessible to the computation module for providing data characterizing the effectiveness of therapeutic agents for the plurality of cell states.

12. The treatment selection system of claim 11 wherein the database is further for providing growth and death rates of the plurality of cell states, and the transition rates between these states.

13. The treatment selection system of claim 10 further comprising a database accessible to the computation module for providing data characterizing the relationships between heritable states and transient functional states, and the probability of a given transient functional state given a specific heritable state.

14. The treatment selection system of claim 10 further comprising a database accessible to the computation module for characterizing the biodistribution pattern of drug concentrations as a function of dose.

**Patentansprüche**

1. Computerimplementiertes Verfahren zur Behandlungsauswahl anhand von genetischer evolutionärer Dynamik und Einzelzellheterogenität, die eine zeitliche Evolution auf der Basis von Übergangsraten zwischen verschiedenen Zelltypen, die durch ihre Resistenz gegen therapeutische Mittel definiert sind, sowie ihrer individuellen Wachstums- und Todesraten charakterisiert, wobei das Verfahren Folgendes beinhaltet:

Akzeptieren von Daten, die Populationen von mehreren heterogenen Zellzuständen zumindest teilweise auf der Basis von Messungen des Krebses eines Subjekts charakterisieren, wobei die mehreren Zustände gemäß ihrer Reaktion auf verschiedene Kombinationen von therapeutischen Mitteln, der Übergangsrate von einem Zelltyp zu einem anderen Zelltyp definiert sind, wobei die mehreren Zellzustände eine Gesamtpopulation aufweisen und die Zellzustände einen ersten Zellzustand mit der größten Population der Zellzustände umfassen;

Akzeptieren einer Spezifikation von jeder von mehreren Behandlungsstrategien, wobei jede Behandlungsstrategie eine Auswahl einer Sequenz und eines Zeitpunkts von dem Subjekt zuzuführenden therapeutischen Mitteln darstellt, wobei mindestens einige der Behandlungsstrategien auf einen anderen Zellzustand als den ersten Zellzustand abzielen, ohne auf den ersten Zellzustand oder die Gesamtpopulation der mehreren Zellzustände abzuzielen;

Bestimmen, mittels einer computerimplementierten Prozedur, einer Wirksamkeit von jeder der mehreren Behandlungsstrategien auf der Basis von erwartetes Wachstum von und den Übergang zwischen den mehreren Zuständen mit der Zuführung verschiedener der therapeutischen Mittel charakterisierenden Daten, einschließlich des Berechnens von Daten, die die Evolution der Populationen der mehreren Zellzustände charakterisieren, wobei die Wirksamkeit einer Behandlungsstrategie aus der Evolution der Populationen der Zellzustände durch Bestimmen des Nettowachstums für einen Zellzustand bestimmt wird, wobei das Nettowachstum für die Zelle vom Typ i dargestellt wird durch:

$$g_0 x_i + \sum_{j \neq i} T(i,j) g_0 x_j - [\sum_k S_a(i,k) d_k] x_i$$

unter der Annahme, dass Zelltodraten bei fehlender Therapie null sind, dass alle Zellen die gleiche Wachstumsrate go haben und dass die therapeutischen Mittel durch Erhöhen von Zelltod wirken, und wobei $x_i$ die Zellpopulation des Zelltyps i darstellt, $T(i, j)$ die Übergangsrate (pro Zelle pro Generation) von Zelltyp j zu i darstellt; $S_a(i, k)$ die Empfindlichkeit von Zelltyp i gegenüber Medikament k darstellt und $d_k$ die normalisierte Dosis von Medikament k darstellt; und
Auswählen einer Behandlungsstrategie gemäß der bestimmten Wirksamkeit.

2.  Verfahren nach Anspruch 1, wobei die Populationen der mehreren Zellzustände charakterisierenden Daten ferner auf Wissensdaten (116) einschließlich probabilistischer Informationen einschließlich nicht erkannter aktueller Zustände oder wahrscheinlicher zukünftiger Zustände basieren.

3.  Verfahren nach Anspruch 1, wobei die Populationen der mehreren Zellzustände charakterisierenden Daten ferner auf einer Messung von Populationen mehrerer Zellzustände in mindestens einem aus einer Gewebeprobe, einer Körperflüssigkeitsprobe und einem Molekularbild beruhen.

4.  Verfahren nach Anspruch 3, wobei das Messen von Populationen mehrerer Zellzustände die Anwendung einer molekularen Messtechnik beinhaltet.

5.  Verfahren nach Anspruch 4, wobei das Anwenden der molekularen Messtechnik das Anwenden einer Polymerase-Kettenreaktionstechnik beinhaltet.

6.  Verfahren nach Anspruch 1, wobei das Bestimmen ferner das Schätzen von Größen jeder Population der mehreren Zellzustände auf der Basis von gemessenen Populationsgrößen der mehreren Zellzustände und von abgeleiteten Populationsgrößen der mehreren Zellzustände beinhaltet.

7.  Verfahren nach Anspruch 1, wobei die erwartetes Wachstum von und den Übergang zwischen den mehreren Zuständen charakterisierenden Daten Daten umfassen, die durch In-vitro- und/oder In-vivo-Experimente bestimmt wurden.

8.  Verfahren nach Anspruch 1, wobei die erwartetes Wachstum von und den Übergang zwischen den mehreren Zuständen charakterisierenden Daten Informationen enthalten, die durch Ex-vivo-Experimente an von Patienten stammendem biologischem Material bestimmt wurden.

9.  Verfahren nach Anspruch 1, wobei jede Behandlungsstrategie eine Auswahl einer Sequenz, eines Zeitpunkts und einer Dosierung von dem Subjekt zuzuführenden therapeutischen Mitteln darstellt.

10. Behandlungsauswahlsystem unter Anwendung von genetischer evolutionärer Dynamik und Einzelzellheterogenität, die eine zeitliche Evolution auf der Basis von Übergangsraten zwischen verschiedenen Zelltypen, die durch ihre Resistenz gegen therapeutische Mittel definiert sind, sowie ihren individuellen Wachstums- und Todesraten charakterisiert, wobei das System Folgendes umfasst:

    einen Eingang zum Akzeptieren von Populationen mehrerer heterogener Zellzustände zumindest teilweise auf der Basis von Messungen des Krebses eines Subjekts charakterisierenden Daten, wobei die mehreren Zustände gemäß ihrer Reaktion auf verschiedene Kombinationen von therapeutischen Mitteln, Übergangsraten von einem Zelltyp zu einem anderen Zelltyp definiert sind, wobei die mehreren Zellzustände eine Gesamtpopulation aufweisen und die Zellzustände einen ersten Zellzustand mit der größten Population der Zellzustände umfassen;
    einen Datenspeicher zum Halten einer Spezifikation von jeder von mehreren Behandlungsstrategien, wobei jede Behandlungsstrategie eine(n) ausgewählte(n) Sequenz und Zeitpunkt für dem Subjekt zuzuführende therapeutische Mittel darstellt, wobei mindestens einige der Behandlungsstrategien auf einen anderen Zellzustand als den ersten Zellzustand abzielen, ohne auf den ersten Zellzustand oder die Gesamtpopulation der mehreren Zellzustände abzuzielen; und

ein Berechnungsmodul, konfiguriert zum Bestimmen einer Wirksamkeit jeder der mehreren Behandlungsstrategien auf der Basis von erwartetes Wachstum von und den Übergang zwischen den mehreren Zuständen mit der Zuführung verschiedener therapeutischer Mittel charakterisierenden Daten, einschließlich des Berechnens von die Evolution der Populationen der mehreren Zellzustände charakterisierenden Daten, wobei die Wirksamkeit einer Behandlungsstrategie aus der Evolution der Populationen der Zellzustände durch Bestimmen des Nettowachstums für einen Zellzustand bestimmt wird, wobei das Nettowachstum für die Zelle vom Typ i dargestellt wird durch:

$$g_0 x_i + \sum_{j \neq i} T(i, j) g_0 x_j - [\sum_k S_a(i, k) d_k)] x_i$$

unter der Annahme, dass Zelltodraten bei fehlender Therapie null sind, dass alle Zellen die gleiche Wachstumsrate go haben und dass die therapeutischen Mittel durch Erhöhen von Zelltod wirken, und wobei $x_i$ die Zellpopulation des Zelltyps i darstellt, $T(i, j)$ die Übergangsrate (pro Zelle pro Generation) von Zelltyp j zu i darstellt; $S_a(i, k)$ die Empfindlichkeit des Zelltyps i gegenüber Medikament k darstellt, und $d_k$ die normalisierte Dosis von Medikament k darstellt; und

zum Auswählen einer Behandlungsstrategie gemäß der bestimmten Wirksamkeit.

11. Behandlungsauswahlsystem nach Anspruch 10, das ferner eine Datenbank umfasst, auf die das Berechnungsmodul zugreifen kann, um die Wirksamkeit von therapeutischen Mitteln für die mehreren Zellzustände charakterisierende Daten bereitzustellen.

12. Behandlungsauswahlsystem nach Anspruch 11, wobei die Datenbank ferner zum Bereitstellen von Wachstums- und Todesraten der mehreren Zellzustände und der Übergangsraten zwischen diesen Zuständen dient.

13. Behandlungsauswahlsystem nach Anspruch 10, das ferner eine Datenbank umfasst, auf die das Berechnungsmodul zugreifen kann, um die Beziehungen zwischen vererbbaren Zuständen und transienten Funktionszuständen und die Wahrscheinlichkeit eines gegebenen transienten Funktionszustands bei einem spezifischen vererbbaren Zustand charakterisierende Daten bereitzustellen.

14. Behandlungsauswahlsystem nach Anspruch 10, das ferner eine Datenbank umfasst, auf die das Berechnungsmodul zugreifen kann, um das Biodistributionsmuster von Medikamentkonzentrationen in Abhängigkeit von der Dosis zu charakterisieren.

**Revendications**

1. Procédé mis en œuvre par ordinateur pour une sélection de traitement à l'aide de dynamique d'évolution génétique et d'hétérogénéité unicellulaire caractérisant une évolution dans le temps sur la base de taux de transition entre différents types de cellules définis par leur résistance à des agents thérapeutiques ainsi que de leurs taux de croissance et de mort individuels, le procédé comprenant :

l'acceptation de données caractérisant des populations d'une pluralité d'états cellulaires hétérogènes au moins en partie sur la base de la mesure du cancer d'un sujet, les états de la pluralité d'états étant définis en fonction de leur réponse à différentes combinaisons d'agents thérapeutiques, du taux de transition d'un type de cellule à un autre type de cellule, la pluralité d'états cellulaires ayant une population globale, et les états cellulaires incluant un premier état cellulaire ayant la plus grande population des états cellulaires ;
l'acceptation d'une spécification de chaque stratégie de traitement d'une pluralité de stratégies de traitement, chaque stratégie de traitement représentant une sélection d'une séquence et d'une synchronisation d'agents thérapeutiques à introduire chez le sujet, où au moins certaines des stratégies de traitement sont ciblées sur un état cellulaire autre que le premier état cellulaire sans cibler le premier état cellulaire ou la population globale de la pluralité d'états cellulaires ;
la détermination à l'aide d'une procédure mise en œuvre par ordinateur d'une efficacité de chaque stratégie de traitement de la pluralité de stratégies de traitement sur la base de données caractérisant la croissance attendue de la pluralité d'états et la transition attendue entre la pluralité d'états avec l'introduction de différents agents des agents thérapeutiques, incluant le calcul de données caractérisant l'évolution des populations de la pluralité d'états cellulaires, où l'efficacité d'une stratégie de traitement est déterminée à partir de l'évolution des populations des états cellulaires, en déterminant la croissance nette pour un état cellulaire, où la croissance nette pour la cellule de type i est représentée par :

$$g_0 x_i + \sum_{j \neq i} T(i,j) g_0 x_j - [\sum_k S_a(i,k) d_k)] x_i$$

en supposant que les taux de mort cellulaire sont nuls en l'absence de thérapie, que toutes les cellules ont le même taux de croissance go, et que les agents thérapeutiques agissent en augmentant la mort cellulaire, et où $x_i$ représente la population cellulaire du type de cellule i, $T(i,j)$ représente le taux de transition (par cellule par génération) du type de cellule j à i ; $S_a(i,k)$ représente la sensibilité du type de cellule i au médicament k, et $d_k$ représente la dose normalisée de médicament k ; et
la sélection d'une stratégie de traitement en fonction de l'efficacité déterminée.

2. Procédé selon la revendication 1, où les données caractérisant les populations de la pluralité d'états cellulaires sont en outre basées sur des données de connaissance (116) incluant des informations probabilistes incluant des états actuels non détectés ou des états futurs probables.

3. Procédé selon la revendication 1, où les données caractérisant les populations de la pluralité d'états cellulaires sont en outre basées sur une mesure de populations d'une pluralité d'états cellulaires dans au moins élément parmi un échantillon de tissu, un échantillon de fluide corporel et une image moléculaire.

4. Procédé selon la revendication 3, où la mesure de populations d'une pluralité d'états cellulaires inclut l'application d'une technique de mesure moléculaire.

5. Procédé selon la revendication 4, où l'application de la technique de mesure moléculaire inclut l'application d'une technique de réaction en chaîne par polymérase.

6. Procédé selon la revendication 1, où la détermination inclut en outre l'estimation de tailles de chaque population de la pluralité d'états cellulaires sur la base de tailles de population mesurées de la pluralité d'états cellulaires et de tailles de population déduites de la pluralité d'états cellulaires.

7. Procédé selon la revendication 1, où les données caractérisant la croissance attendue de la pluralité d'états et la transition attendue entre la pluralité d'états incluent des données déterminées par une expérimentation in vitro et/ou in vivo.

8. Procédé selon la revendication 1, où les données caractérisant la croissance attendue de la pluralité d'états et la transition attendue entre la pluralité d'états incluent des informations déterminées par une expérimentation ex vivo sur un matériau biologique dérivé d'un patient.

9. Procédé selon la revendication 1, où chaque stratégie de traitement représente une sélection d'une séquence, d'une synchronisation et d'un dosage d'agents thérapeutiques à introduire chez le sujet.

10. Système de sélection de traitement à l'aide de dynamique d'évolution génétique et d'hétérogénéité unicellulaire caractérisant une évolution dans le temps sur la base de taux de transition entre différents types de cellules définis par leur résistance à des agents thérapeutiques ainsi que de leurs taux de croissance et de mort individuels, le système comprenant :

une entrée pour accepter des données caractérisant des populations d'une pluralité d'états cellulaires hétérogènes au moins en partie sur la base de la mesure du cancer d'un sujet, les états de la pluralité d'états étant définis en fonction de leur réponse à différentes combinaisons d'agents thérapeutiques, du taux de transition d'un type de cellule à un autre type de cellule, la pluralité d'états cellulaires ayant une population globale, et les états cellulaires incluant un premier état cellulaire ayant la plus grande population des états cellulaires ;

un stockage de données pour contenir une spécification de chaque stratégie de traitement d'une pluralité de stratégies de traitement, chaque stratégie de traitement représentant une séquence et une synchronisation sélectionnées d'agents thérapeutiques à introduire chez le sujet, où au moins certaines des stratégies de traitement sont ciblées sur un état cellulaire autre que le premier état cellulaire sans cibler le premier état cellulaire ou la population globale de la pluralité d'états cellulaires ; et

un module de calcul configuré pour déterminer une efficacité de chaque stratégie de traitement de la pluralité de stratégies de traitement sur la base de données caractérisant la croissance attendue de la pluralité d'états et la transition attendue entre la pluralité d'états avec l'introduction de différents agents des agents thérapeutiques, incluant le calcul de données caractérisant l'évolution des populations de la pluralité d'états cellulaires, où l'efficacité d'une stratégie de traitement est déterminée à partir de l'évolution des populations des états cellulaires, en déterminant la croissance nette pour un état cellulaire, où la croissance nette pour la cellule de type i est représentée par :

$$g_0 x_i + \sum_{j \neq i} T(i,j) g_0 x_j - \left[\sum_k S_a(i,k) d_k\right] x_i$$

en supposant que les taux de mort cellulaire sont nuls en l'absence de thérapie, que toutes les cellules ont le même taux de croissance go, et que les agents thérapeutiques agissent en augmentant la mort cellulaire, et où $x_i$ représente la population cellulaire du type de cellule i, $T(i,j)$ représente le taux de transition (par cellule par génération) du type de cellule j à i ; $S_a(i,k)$ représente la sensibilité du type de cellule i au médicament k, et $d_k$ représente la dose normalisée du médicament k ; et

pour sélectionner une stratégie de traitement en fonction de l'efficacité déterminée.

11. Système de sélection de traitement selon la revendication 10, comprenant en outre une base de données accessible au module de calcul pour fournir des données caractérisant l'efficacité d'agents thérapeutiques pour la pluralité d'états cellulaires.

12. Système de sélection de traitement selon la revendication 11, où la base de données est en outre destinée à fournir des taux de croissance et de mort de la pluralité d'états cellulaires, et les taux de transition entre ces états.

13. Système de sélection de traitement selon la revendication 10, comprenant en outre une base de données accessible au module de calcul pour fournir des données caractérisant les relations entre des états héréditaires et des états fonctionnels transitoires, et la probabilité d'un état fonctionnel transitoire donné étant donné un état héréditaire spécifique.

14. Système de sélection de traitement selon la revendication 10, comprenant en outre une base de données accessible au module de calcul pour caractériser le motif de biodistribution de concentrations de médicament en fonction de la dose.

102 — Biopsy

104 — Cell Population Measurement Device

106 — Measured Cell Populations

Probability Information
- Undetected Current States
- Probable Future States

Empirical Data Regarding Cancer Evolution

116

Cell Population Estimator

108 —

110 — Simulation Initial Conditions

112 — Simulator

114 — Simulation Results

Evaluate

FIG. 1

100

200

FIG. 2

FIG. 3

FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009155009 A

**Non-patent literature cited in the description**

- **ROBERT A. BECKMAN et al.** Impact of genetic dynamics and single-cell heterogeneity on development of nonstandard personalized medicine strategies for cancer. *Proc. National Academy of Science of the U.S.A.,* 04 September 2012, vol. 109 (36), 14586-91